(19) Europäisches Patentamt
European Patent Office
Office européen des brevets

(11) **EP 4 026 492 A1**

(12) **EUROPEAN PATENT APPLICATION**
published in accordance with Art. 153(4) EPC

(43) Date of publication:
**13.07.2022 Bulletin 2022/28**

(21) Application number: **19944607.1**

(22) Date of filing: **01.11.2019**

(51) International Patent Classification (IPC):
***A61B 5/026*** (2006.01)

(52) Cooperative Patent Classification (CPC):
**A61B 6/504; A61B 5/02007; A61B 5/0215;**
**A61B 6/481; A61B 6/507; G06T 7/0016;**
A61B 6/466; G06T 2207/10016; G06T 2207/20036;
G06T 2207/20224; G06T 2207/30021;
G06T 2207/30048; G06T 2207/30101;
G06T 2207/30104; G06T 2207/30172

(86) International application number:
**PCT/CN2019/115043**

(87) International publication number:
**WO 2021/042479 (11.03.2021 Gazette 2021/10)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **05.09.2019 CN 201910835038**

(71) Applicant: **Suzhou Rainmed Medical Technology Co., Ltd.**
**Suzhou, Jiangsu 215000 (CN)**

(72) Inventors:
• **LIU, Guangzhi**
  **Suzhou, Jiangsu 215000 (CN)**

• **WANG, Zhiyuan**
  **Suzhou, Jiangsu 215000 (CN)**
• **GU, Yifan**
  **Suzhou, Jiangsu 215000 (CN)**
• **WANG, Zhiting**
  **Suzhou, Jiangsu 215000 (CN)**
• **HUO, Yong**
  **Suzhou, Jiangsu 215000 (CN)**
• **GONG, Yanjun**
  **Suzhou, Jiangsu 215000 (CN)**
• **LI, Jianping**
  **Suzhou, Jiangsu 215000 (CN)**
• **YI, Tieci**
  **Suzhou, Jiangsu 215000 (CN)**

(74) Representative: **Boult Wade Tennant LLP**
**Salisbury Square House**
**8 Salisbury Square**
**London EC4Y 8AP (GB)**

(54) **METHOD, DEVICE AND SYSTEM FOR CONVENIENTLY AND QUICKLY MEASURING ASSESSMENT PARAMETER FOR CORONARY ARTERY**

(57) A method, an apparatus and a system for conveniently measuring coronary artery vascular evaluation parameters are provided by the disclosure. The measurement method includes: measuring a pressure $P_d$ at a distal end of coronary artery stenosis and/or a pressure $P_a$ at a coronary artery inlet via a pressure guide wire (S100); performing coronary angiography for a blood vessel to be measured (S200); selecting an angiogram image of a first body position and an angiogram image of a second body position of the blood vessel to be measured (S300); selecting a segment of blood vessel from a proximal end to a distal end of the coronary artery for segmentation, and obtaining a three-dimensional coronary artery vascular model by three-dimensional modeling (S400); injecting a contrast agent, and obtaining an average time $T_a$ taken for the contrast agent passing from an inlet to an outlet of the segment of blood vessel (S500); obtaining a time $T_{max}$ taken for the contrast agent passing from the inlet to the outlet of the segment of blood vessel in a maximum dilated state according to hydrodynamic formulas (S600); obtaining coronary artery vascular evaluation parameters (S700). The measurement method is able to perform modeling based on angiogram images at any time in the cardiac cycle, making the three-dimensional modeling convenient, and shortening the injection time of the vasodilator. The measurement process is simple, and the measurement result is accurate.

EP 4 026 492 A1

S100 — Measuring a pressure $P_d$ at a distal end of coronary artery stenosis and/or a pressure $P_a$ at a coronary artery inlet via a pressure guide wire;

S200 — Performing coronary angiography for a blood vessel to be measured;

S300 — Selecting, in a resting state, an angiogram image of a first body position and an angiogram image of a second body position of the blood vessel to be measured;

S400 — Selecting a segment of blood vessel from a proximal end to a distal end of the coronary artery for segmentation, and obtaining a three-dimensional coronary artery vascular model by three-dimensional modeling based on the angiogram image of the first body position and the angiogram image of the second body position;

S500 — Injecting a contrast agent, and obtaining an average time $T_a$ taken for the contrast agent passing from an inlet to an outlet of the segment of blood vessel according to the three-dimensional coronary artery vascular model;

S600 — Obtaining a time $T_{max}$ taken for the contrast agent passing from the inlet to the outlet of the segment of blood vessel in a maximum dilated state according to the three-dimensional coronary artery vascular model and hydrodynamic formulas;

S700 — Obtaining coronary artery vascular evaluation parameters based on $T_a$, $T_{max}$ and/or $P_d$ and/or $P_a$.

FIG.1

# Description

The disclosure claims priority to Chinese Patent Application No. 201910835038.3 filed before Chinese National Intellectual Property Administration on September 5, 2019, entitled "METHOD, APPARATUS AND SYSTEM FOR CONVENIENTLY MEASURING CORONARY ARTERY VASCULAR EVALUATION PARAMETERS", the entire contents of which are hereby incorporated by reference.

## TECHNICAL FIELD

The present disclosure relates to the field of coronary artery medical technology, and in particular to, a method and an apparatus for conveniently measuring coronary artery vascular evaluation parameters, a coronary artery analysis system and a computer storage medium.

## BACKGROUND

The heart is a high energy consumption organ. In a resting state, the oxygen uptake of myocardial metabolism can reach 60% to 80% of the blood oxygen content. Therefore, under stress conditions such as exercise, it is difficult for the heart to meet the increased demand for myocardial hypoxia by increasing the oxygen uptake capacity of the tissue, and in most circumstances, the demand is met by increasing the myocardial blood flow to ensure the oxygen demand for myocardial metabolism. Myocardial microcirculation occupies 95% of the coronary artery circulation, which plays a role in regulating myocardial blood flow through various factors such as local metabolites, endothelium, neuroendocrine, and myogenicity. Studies have shown that abnormal coronary artery microcirculation is an important predictor of poor long-term prognosis in patients suffering from coronary heart disease.

In 2013, the guidelines were changed, which stated "for patients with suspected microvascular angina, if there is no obvious abnormality in coronary angiography, intracavitary injection of acetylcholine or adenosine for Doppler measurement can be considered during the angiography, to calculate endothelial dependent or non-endothelial dependent CFR, and to determine whether there is microcirculation/epicardial vascular spasm", which is provided as a category IIB recommendation.

In 2019, the guidelines have added 1 category IIA recommendation and 2 category IIB recommendations. It is proposed that "for patients with persistent symptoms but coronary angiography being normal or showing moderate stenosis with preserved iwfr/FFR values, it should consider using microcirculatory resistance measurement and/or CFR based on guide wire measurement", which is provided as a category IIA recommendation.

Coronary artery microvascular function is accomplished by detecting the response of capillaries to a vasodilator. The changes in these two aspects of the guidelines also indicate the importance of coronary artery microvascular function tests. The measurement indicator used for coronary artery microvascular function refers to the maximum dilated degree of coronary capillaries, that is, coronary flow reserve (CFR). The vasodilators used mainly comprise non-endothelium independent vasodilators acting on vascular smooth muscle and endothelium dependent vasodilators acting on vascular endothelial cells, including adenosine and acetylcholine.

For patients with no obvious stenosis on coronary angiography but suspected of coronary artery disease (CAD), our previous examination means was injection of adenosine and acetylcholine to detect the response of capillaries to vasodilators. Current examination means mainly comprise coronary fractional flow reserve (FFR) and index of microcirculatory resistance (IMR). Regarding the IMR, by synchronously recording the coronary pressure and temperature with a soft pressure guide wire, the transit mean time (Tmn) taken for saline flowing from a guiding catheter to a temperature receptor at the tip of the guide wire can be known from the time differences between temperature changes detected by the two temperature receptors on the guide wire bar. An IMR value can be obtained according to the product of the pressure at a distal end of the coronary artery Pd and Tmn. But, as a whole, there are not many methods to evaluate microcirculation. Existing examination means simplifies the process, improves safety, and optimizes the results. Therefore, the recommendation level of the guideline has been improved compared to before. In addition, non-invasive examinations including transthoracic Doppler ultrasound, radionuclide imaging technology, and nuclear magnetic resonance imaging technology are valuable in the diagnosis of microcirculation diseases, but they all have varying degrees of deficiencies and still have not become recommended methods for microcirculation function evaluation.

Existing CFR measurement methods comprise: (1) Doppler guide wire measurement method, in which the Doppler guide wire is delivered into the coronary artery (distal to the lesion) to directly measure blood flow velocity in the coronary artery in a resting state and in a maximum hyperemia state, and then CFR can be calculated; and (2) the thermodilution curve measurement method, in which the temperature change in the coronary artery can be sensed directly via the dual-sensing guide wire embedded with temperature-pressure receptor, and the thermodilution curve in the coronary artery in the resting state and in the maximum hyperemia state can be obtained, then it can use the transit mean time of blood flow instead of the coronary artery flow velocity to calculate the CFR.

Measuring the IMR and CFR by the pressure guide wire sensor has the following problems. (1) If the pressure guide wire sensor is too close to the coronary artery inlet, the measured Tmn will be too small, resulting in relative minor IMR result, and if too far from the coronary artery inlet, the measured Tmn will be too large, resulting in relative large IMR result. (2) Since it is necessary to inject normal saline for total 6 times in the resting state and the maximum hyperemia state, if the position of the pressure guide wire sensor shifts, then respective measurement results will be not comparable, and the measurement process is cumbersome. (3) The obtained Tmn results could be quite different for each injection of saline, and if a value of Tmn for an individual injection differs from other 2 values by more than 30%, it is necessary to inject the saline again for measurement, increasing the number of saline injections. (4) If the temperature of the injected saline does not drop rapidly enough for the pressure guide wire receptor to detect, there will be a failure to record. Therefore, many confounding variables are present. It is noted that there is a need for increased injection velocity, increased volume, and decreased temperatures. (5) Errors may occur if the temperature does not return back to the initial value within a given interval (0.6s). This may be due to slow injection, uneven injection, or injection volume too high and the like. Therefore, the distance of the pressure guide wire receptor, the injection speed of saline, the injection volume, and the temperature of the saline will directly affect the measurement results, causing inaccurate results and redundant procedures. Moreover, prolonged and continuous injections of vasodilators will cause severe discomfort.

## SUMMARY

The present disclosure provides a method and an apparatus for conveniently measuring coronary artery vascular evaluation parameters, a coronary artery analysis system and a computer storage medium, so as to solve the problems in the prior art, i.e., when measuring CFR and IMR by use of a pressure guide wire, patients are heavily affected and suffer from severe discomfort due to long-term continuous injections of vasodilators, as well as cumbersome measurement process with the pressure guide wire, and inaccurate measurement results.

In order to achieve the foregoing objectives, in a first aspect, the disclosure provides a method for conveniently measuring coronary artery vascular evaluation parameters, comprising:

measuring a pressure $P_d$ at a distal end of coronary artery stenosis and/or a pressure $P_a$ at a coronary artery inlet via a pressure guide wire;
performing coronary angiography for a blood vessel to be measured;
selecting an angiogram image of a first body position and an angiogram image of a second body position of the blood vessel to be measured;
selecting a segment of blood vessel from a proximal end to a distal end of the coronary artery for segmentation, and obtaining a three-dimensional coronary artery vascular model by three-dimensional modeling based on the angiogram image of the first body position and the angiogram image of the second body position;
injecting a contrast agent, and obtaining an average time $T_a$ taken for the contrast agent passing from an inlet to an outlet of the segment of blood vessel according to the three-dimensional coronary artery vascular model;
obtaining a time $T_{max}$ taken for the contrast agent passing from the inlet to the outlet of the segment of blood vessel in a maximum dilated state according to the three-dimensional coronary artery vascular model and hydrodynamic formulas;
obtaining coronary artery vascular evaluation parameters based on $T_a$, $T_{max}$ and/or $P_d$ and/or $P_a$.

Optionally, in the above method for conveniently measuring the coronary artery vascular evaluation parameters, the coronary artery vascular evaluation parameters comprise coronary flow reserve CFR and an index of microcirculatory resistance IMR as coronary artery blood flow increases from a resting state to a hyperemic state.

Optionally, in the above method for conveniently measuring the coronary artery vascular evaluation parameters, the CFR = $T_a/T_{max}$; and/or the IMR = $P_d \times T_{max}$.

Optionally, in the above method for conveniently measuring the coronary artery vascular evaluation parameters, obtaining an average time $T_a$ taken for the contrast agent passing from an inlet to an outlet of the segment of blood vessel comprises:

obtaining a time $T_1$ taken for the contrast agent passing from an inlet to an outlet of the segment of blood vessel within the angiogram image of the first body position and obtaining a time $T_2$ taken for the contrast agent passing from an inlet to an outlet of the segment of blood vessel within the angiogram image of the second body position,

$$T_a = \frac{\left(T_1 + T_2\right)}{2}.$$

Optionally, in the above method for conveniently measuring the coronary artery vascular evaluation parameters, the time $T_1$ and the time $T_2$ are calculated according to a ratio of the number of frames of partial area images divided by a heartbeat cycle area to the number of frames transmitted per second.

Optionally, in the above method for conveniently measuring the coronary artery vascular evaluation parameters, obtaining a time $T_{max}$ taken for the contrast agent passing from the inlet to the outlet of the segment of blood vessel in the maximum dilated state comprises:

measuring a length L of the selected segment of the blood vessel;
deriving a blood flow velocity V in a dilated state by means of the hydrodynamic calculating method according to the three-dimensional coronary artery vascular model, $P_a$ and $P_d$;
obtaining the time $T_{max}$ taken for the contrast agent passing from the inlet to the outlet of the segment of blood vessel in the maximum dilated state according to the formula $T_{max} = L/V$.

Optionally, in the above method for conveniently measuring the coronary artery vascular evaluation parameters, deriving a blood flow velocity V in the dilated state by means of the hydrodynamic calculating method according to the three-dimensional coronary artery vascular model, $P_a$ and $P_d$ comprises:

obtaining the diameter Dt of the segment of blood vessel at a time interval of t;
obtaining the pressure $P_d$ at the distal end of coronary artery stenosis and the pressure $P_a$ at the coronary artery inlet via the pressure guide wire at the time interval of t;
calculating a FFRt value at the time interval of t according to Dt, $P_a$, and $P_d$, and obtaining a blood flow velocity Vt by inverse calculation according to the definition of FFR;
injecting a vasodilator and measuring the FFR value in the dilated state; comparing the FFR value in the dilated state with the real-time FFRt value to obtain the blood flow velocity Vt correspondingly, namely the blood flow velocity V in the dilated state.

Optionally, in the above method for conveniently measuring the coronary artery vascular evaluation parameters, an angle between the first body position and the second body position is greater than 30°

Optionally, in the above method for conveniently measuring the coronary artery vascular evaluation parameters, obtaining a three-dimensional coronary artery vascular model by three-dimensional modeling based on the angiogram image of the first body position and the angiogram image of the second body position comprises:

removing an interfering blood vessel from the angiogram image of the first body position and the angiogram image of the second body position to obtain a result image;
extracting a centerline and diameter of the coronary artery from each result image along an extension direction of the coronary artery;
projecting the centerline and diameter of each coronary artery onto a three-dimensional space for three-dimensional modeling to obtain a three-dimensional coronary artery vascular model.

In a second aspect, the present disclosure provides an apparatus for conveniently measuring coronary artery vascular evaluation parameters, for use in the above method for conveniently measuring the coronary artery vascular evaluation parameters, comprising: a pressure guide wire measurement unit, a coronary angiography extraction unit, a three-dimensional modeling unit and a parametric measurement unit. The coronary angiography extraction unit is connected to the three-dimensional modeling unit. The parametric measurement unit is connected to the pressure guide wire measurement unit and the three-dimensional modeling unit.

The pressure guide wire measurement unit is configured to measure a pressure $P_d$ at a distal end of coronary artery stenosis and a pressure $P_a$ at a coronary artery inlet via the pressure guide wire.

The coronary angiography extraction unit is configured to select an angiogram image of a first body position and an angiogram image of a second body position of the blood vessel to be measured.

The three-dimensional modeling unit is configured to receive the angiogram image of the first body position and the angiogram image of the second body position transmitted by the coronary angiography extraction unit and to three-dimensionally model so as to obtain a three-dimensional coronary artery vascular model.

The parametric measurement unit is configured to receive the three-dimensional coronary artery vascular model transmitted by the three-dimensional modeling unit to obtain an average time $T_a$ taken for a contrast agent passing from an inlet to an outlet of a segment of blood vessel; and obtaining a time $T_{max}$ taken for the contrast agent passing from the inlet to the outlet of the segment of blood vessel in a maximum dilated state according to the three-dimensional coronary artery vascular model and hydrodynamic formulas; obtaining coronary artery vascular evaluation parameters based on $T_a$, $T_{max}$ and/or $P_d$ and/or $P_a$.

Optionally, in the above apparatus for conveniently measuring the coronary artery vascular evaluation parameters, the parametric measurement unit comprises: a coronary flow reserve module, a module for index of microcirculatory resistance, and/or a coronary fractional flow reserve module. The coronary flow reserve module and the module for index of microcirculatory resistance are connected to the three-dimensional modeling unit. The module for index of microcirculatory resistance and the coronary fractional flow reserve module are connected to the pressure guide wire measurement unit.

The coronary flow reserve module is configured to measure the coronary flow reserve CFR as the coronary artery blood flow increases from a resting state to a hyperemic state, CFR= $T_a/T_{max}$.

The module for index of microcirculatory resistance is configured to measure the index of microcirculatory resistance IMR, IMR = $P_d \times T_{max}$.

The coronary fractional flow reserve module is configured to measure the coronary fractional flow reserve FFR, FFR=$P_d/P_a$.

In a third aspect, the present disclosure provides a coronary artery analysis system comprising an apparatus for conveniently measuring the coronary artery vascular evaluation parameters according to claim 10 or 11.

In a fourth aspect, the present disclosure provides a computer storage medium, wherein the above method for conveniently measuring the coronary artery vascular evaluation parameters is implemented when a computer program is executed by a processor.

The beneficial effects of the solutions provided by the embodiments of the present disclosure comprise at least:

The disclosure provides a method for conveniently measuring coronary artery vascular evaluation parameters, which is able to perform modeling based on angiogram images at any time in the cardiac cycle, making the three-dimensional modeling convenient. Injection of a vasodilator is only required when measuring FFR in a dilated state, but in other procedures there is no need for injection of the vasodilator, greatly shortening the injection time of the vasodilator. Next, the coronary angiogram image is subjected to three-dimensional modeling to obtain the average time $T_a$ taken for the contrast agent passing from an inlet to an outlet of the segment of blood vessel according to the three-dimensional coronary artery vascular model. A time $T_{max}$ taken for the contrast agent passing from the inlet to the outlet of the segment of blood vessel in a maximum dilated state is obtained according to the three-dimensional coronary artery vascular model and hydrodynamic formulas. Coronary artery vascular evaluation parameters such as IMR and CFR are measured based on $T_a$, $T_{max}$ and/or $P_d$ and/or $P_a$. The measurement process is simple and the measurement results are accurate, overcoming the problems caused by using the pressure guide wires to measure the coronary artery vascular evaluation parameters such as IMR and CFR.

BRIEF DESCRIPTION OF THE DRAWINGS

The drawings illustrated here are used to provide a further understanding of the present disclosure and constitute a part of the present disclosure. The exemplary embodiments and the descriptions thereof are used to explain the present disclosure, and do not constitute an improper limitation on the present disclosure. In the drawings:

FIG. 1 is a flowchart of an embodiment of a method for conveniently measuring coronary artery vascular evaluation parameters according to the disclosure;
FIG. 2 is a flowchart of another embodiment of a method for conveniently measuring coronary artery vascular evaluation parameters according to the disclosure;
FIG. 3 is a flowchart of step S400 of the disclosure;
FIG. 4 is a flowchart of step S600 of the disclosure;
FIG. 5 is a flowchart of step S620 of the disclosure;
FIG. 6 is a structural block diagram of an apparatus for conveniently measuring coronary artery vascular evaluation parameters of the disclosure;
FIG. 7 is a structural block diagram of the parameter measurement unit of the disclosure;
FIG. 8 is a structural block diagram of an embodiment of a three-dimensional modeling unit of the disclosure;
FIG. 9 is a structural block diagram of another embodiment of a three-dimensional modeling unit of the disclosure;
FIG. 10 is a structural block diagram of an image processing module of the disclosure;
FIG. 11 is a reference image;
FIG. 12 is a target image to be segmented;
FIG. 13 is another target image to be segmented;
FIG. 14 is an enhanced catheter image;
FIG. 15 is a binarized image of feature points of a catheter;
FIG. 16 is an enhanced target image;
FIG. 17 is an image of the region where the coronary artery locates;
FIG. 18 is a result image;
FIG. 19 shows angiogram images of two body positions;
FIG. 20 is a diagram of a three-dimensional coronary artery vascular model generated by combining the body position angle and the centerline of the coronary artery with FIG. 19;

The reference signs are described below:
pressure guide wire measurement unit 110, coronary angiography extraction unit 120, three-dimensional modeling unit 130, image-reading module 131, segmentation module 132, blood vessel length measurement module 133, three-dimensional modeling module 134, image processing module 135, image denoising module 1350, catheter feature point extraction module 1351, coronary artery extraction module 1352, coronary centerline extraction module 136, blood vessel diameter measurement module 137, parametric measurement unit 140, coronary flow reserve module 141, module for index of microcirculatory resistance 142, and coronary fractional flow reserve module 143.

DETAILED DESCRIPTION OF PREFERRED EMBODIMENTS

In order to make the objectives, technical solutions and advantages of the disclosure more clear, the technical solutions of the disclosure will be concisely and completely described below with reference to the specific embodiments and corresponding drawings. It is apparent that the described embodiments are merely part of the embodiments of the disclosure rather than all of them. Based on the embodiments in the disclosure, without making creative work, all the other embodiments obtained by a person skilled in the art will fall into the protection scope of the disclosure.

Hereinafter, a plurality of embodiments of the present disclosure will be disclosed with drawings. For clear illustration, many practical details will be described in the following description. However, it should be understood that the present disclosure should not be limited by these practical details. In other words, in some embodiments of the present disclosure, these practical details are unnecessary.

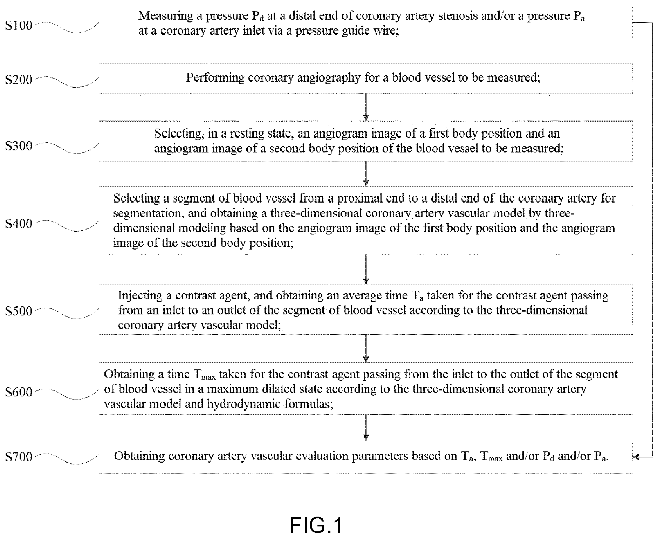

As shown in FIG. 1, the disclosure provides a method for conveniently measuring coronary artery vascular evaluation parameters, comprising:

S100: measuring a pressure $P_d$ at a distal end of coronary artery stenosis and/or a pressure $P_a$ at a coronary artery inlet via a pressure guide wire;

S200: performing coronary angiography for a blood vessel to be measured;

S300: selecting, in a resting state, an angiogram image of a first body position and an angiogram image of a second body position of the blood vessel to be measured;

S400: selecting a segment of blood vessel from a proximal end to a distal end of the coronary artery for segmentation, and obtaining a three-dimensional coronary artery vascular model by three-dimensional modeling based on the angiogram image of the first body position and the angiogram image of the second body position;

S500: injecting a contrast agent, and obtaining an average time $T_a$ taken for the contrast agent passing from an inlet to an outlet of the segment of blood vessel according to the three-dimensional coronary artery vascular model;

S600: obtaining a time $T_{max}$ taken for the contrast agent passing from the inlet to the outlet of the segment of blood vessel in a maximum dilated state according to the three-dimensional coronary artery vascular model and hydrodynamic formulas;

S700: obtaining coronary artery vascular evaluation parameters based on $T_a$, $T_{max}$ and/or $P_d$ and/or $P_a$.

In an embodiment of the present disclosure, the coronary artery vascular evaluation parameters in S700 comprise: a coronary flow reserve CFR and an index of microcirculatory resistance IMR as coronary artery blood flow increases from a resting state to a hyperemic state. In an embodiment of the present disclosure, CFR= $T_a/T_{max}$; IMR= $P_d \times T_{max}$.

The disclosure provides a method for conveniently measuring coronary artery vascular evaluation parameters. Injection of a vasodilator is only required when measuring FFR in a dilated state, but in other procedures there is no need for injection of the vasodilator, greatly shortening the injection time of the vasodilator. Next, the coronary angiogram image is subjected to three-dimensional modeling to obtain the average time $T_a$ taken for the contrast agent passing from the inlet to the outlet of the segment of blood vessel according to the three-dimensional coronary artery vascular model. The time $T_{max}$

for the contrast agent passing from the inlet to the outlet of the segment of blood vessel in a maximum dilated state is obtained according to the three-dimensional coronary artery vascular model and hydrodynamic formulas. Coronary artery vascular evaluation parameters such as IMR and CFR are measured based on $T_a$, $T_{max}$ and/or $P_d$ and/or $P_a$. The measurement process is simple and the measurement results are accurate, overcoming the problems caused by using the pressure guide wires to measure the coronary artery vascular evaluation parameters such as IMR and CFR.

It should be noted that the injection of the vasodilators comprises: intravenous or intracoronary injection of the vasodilator. Injection methods which include using a mixed solution of the vasodilator and contract agent, or subsequently injecting in portions while alternating between both, all fall within the protection scope of the present disclosure. All vasodilating agents including adenosine and ATP are protected by this disclosure as well.

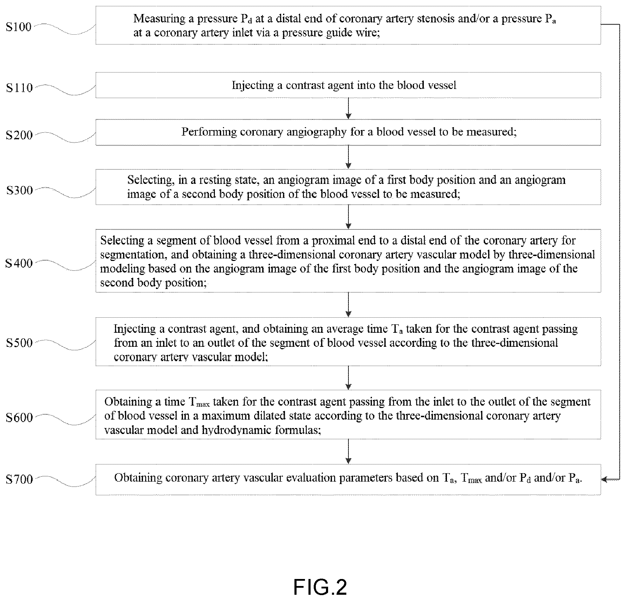

As shown in FIG. 2, an embodiment of the present disclosure, after S100 and before S200, comprises S110: injecting a contrast agent into the blood vessel.

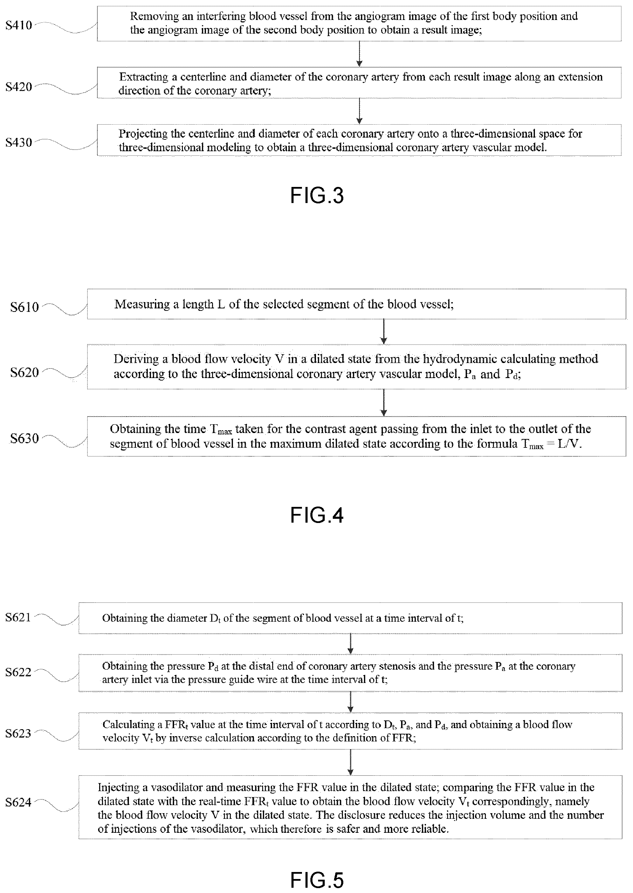

As shown in FIG. 3, in an embodiment of the present disclosure, S400 comprises:

S410: removing an interfering blood vessel from the angiogram image of the first body position and the angiogram image of the second body position to obtain a result image, specifically:

removing an interfering blood vessel from the angiogram image of the first body position and the angiogram image of the second body position;

denoising the coronary angiogram images, comprising static noise removal and dynamic noise removal;

defining a first frame of a segmented image where a catheter appears as a reference image, and defining a k-th frame of the segmented image where a complete coronary artery appears as a target image, wherein k is a positive integer greater than 1;

subtracting the target image from the reference image to extract a feature point O of the catheter, with specific manner of: subtracting the target image from the reference image; denoising, comprising static noise removal and dynamic noise removal; subjecting the denoised image to image enhancement; subjecting the enhanced catheter image to binarization processing to obtain a binarized image with a set of feature points O of the catheter;

subtracting the reference image from the target image to extract an image of region where the coronary artery locates, with specific manner of:

subtracting the reference image from the target image; denoising, comprising static noise removal and dynamic noise removal; subjecting the denoised image to image enhancement; according to a positional relationship between each region in the enhanced target image and the feature points of the catheter, determining and extracting the region of the coronary artery, that is, the image of region where the coronary artery locates;

obtaining the result image based on the image of region using the feature points of the catheter as seed points for dynamic growth, with specific manner of: subjecting the image of region where the coronary artery locates to binarization processing to obtain a binarized coronary arterial image; subjecting the binarized coronary artery image to morphological operations, and carrying out dynamic region growth of the binarized coronary artery image, by using the feature points of the catheter as seed points, according to the position at which the seed points are located to obtain the result image;

S420: extracting a centerline and diameter of the coronary artery from each result image along an extension direction of the coronary artery;

S430: projecting the centerline and diameter of each coronary artery onto a three-dimensional space for three-dimensional modeling to obtain a three-dimensional coronary artery vascular model, with specific manner of:

acquiring the body position angle for taking each of the coronary angiogram images;
projecting the centerline of each coronary artery in combination with the body position angle onto a three-dimensional space, and performing projection to generate the three-dimensional coronary artery vascular model.

In an embodiment of the present disclosure, S500 comprises: obtaining a time $T_1$ taken for the contrast agent passing from an inlet to an outlet of the segment of blood vessel in the angiogram image of the first body position and obtaining a time $T_2$ taken for the contrast agent passing from an inlet to an outlet of the segment of blood vessel in the angiogram image of the second body position,

$$T_a = \frac{(T_1 + T_2)}{2}$$

; the time $T_1$ and the time $T_2$ are calculated according to a ratio of the number of frames of partial area images divided by a heartbeat cycle area to the number of frames transmitted per second; namely: T=N/*fps*, wherein N represents the number of frames of partial area images divided by the heartbeat cycle area, fps represents the number of frames played per second, which generally refers to the number of animation or video frames, T represents a time T taken for the contrast agent passing from the inlet to the outlet of the segment of blood vessel in the angiogram image of a certain body position, and thus $T_1$ and $T_2$ can be calculated according to the above formula. In an embodiment of the present disclosure, *fps*=10~30; preferably, fps=15.

Since $T_1$ and $T_2$ are measured based on the three-dimensional coronary artery vascular model obtained from the angiogram images, the CFR is also measured via the three-dimensional coronary artery vascular model without relying on a pressure guide wire sensor, which overcomes the problem that the pressure guide wire sensor tends to move under the impact of the saline and measures inaccurately, and there is no need to inject saline when the measurement is based on angiogram images, thereby avoiding the influences of the saline injection speed, injection volume, and temperature of the saline on the CFR measurement results and thus improving the accuracy of the measurement.

The IMR measurement is based on the pressure guide wire and the angiogram images. Due to the increase in accuracy of the pressure $P_d$ at the distal end of the coronary artery stenosis measured by the pressure guide wire, and the measurement of the time $T_2$ based on the angiogram images, there is a reduction in vasodilator injection time, saline injection amount, impact of saline on test result, as well as an increase in IMR measurement result accuracy, which in turn simplified the process.

As shown in FIG. 4, in an embodiment of the present disclosure, S600 comprises:

S610: measuring a length L of the selected segment of the blood vessel;
S620: deriving a blood flow velocity V in a dilated state by means of the hydrodynamic calculating method according to the three-dimensional coronary artery vascular model, $P_a$ and $P_d$;
S630: obtaining the time $T_{max}$ taken for the contrast agent passing from the inlet to the outlet of the segment of blood vessel in the maximum dilated state according to the formula $T_{max}$ = L/V.

As shown in FIG. 5, in an embodiment of the present application, the manner of S620 comprises:

S621: obtaining the diameter Dt of the segment of blood vessel at a time interval of t;
S622: obtaining the pressure $P_d$ at the distal end of coronary artery stenosis and the pressure $P_a$ at the coronary artery inlet via the pressure guide wire at the time interval of t;
S623: calculating a FFRt value at the time interval of t according to Dt, $P_a$, and $P_d$, and obtaining a blood flow velocity Vt by inverse calculation according to the definition of FFR;

S624: injecting a vasodilator and measuring the FFR value in the dilated state; comparing the FFR value in the dilated state with the real-time FFRt value to obtain the blood flow velocity Vt correspondingly, namely the blood flow velocity V in the dilated state. The disclosure reduces the injection volume and the number of injections of the vasodilator, which therefore is safer and more reliable.

In an embodiment of the disclosure, the coronary artery vascular evaluation parameters in S600 comprise: a coronary artery fractional flow reserve FFR, $FFR=P_d/P_a$.

In an embodiment of the present disclosure, in S300, an angle between the first body position selected in the image of the first body position and the second body position selected in the image of the second body position is greater than 30°. In this disclosure, images of any two body positions with an angle greater than 30° can be selected for three-dimensional reconstruction, thereby reducing the difficulty in taking images of body positions, simplifying the process, and making modeling convenient and quick.

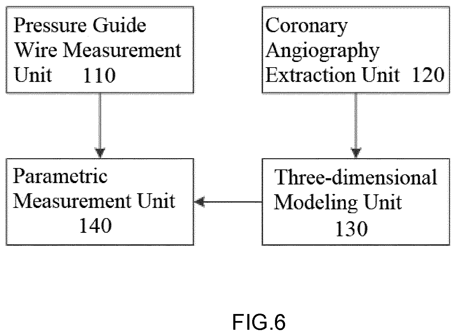

As shown in FIG.6, the present disclosure provides an apparatus for conveniently measuring coronary artery vascular evaluation parameters, for use in the method for conveniently measuring coronary artery vascular evaluation parameters, comprising: a pressure guide wire measurement unit 110, a coronary angiography extraction unit 120, a three-dimensional modeling unit 130 and a parametric measurement unit 140. The coronary angiography extraction unit 120 is connected to the three-dimensional modeling unit 130. The parametric measurement unit 140 is connected to the pressure guide wire measurement unit 110 and the three-dimensional modeling unit 130. The pressure guide wire measurement unit 110 is configured to measure a pressure $P_d$ at a distal end of coronary artery stenosis and a pressure $P_a$ at a coronary artery inlet via the pressure guide wire. The coronary angiography extraction unit 120 is configured to select an angiogram image of a first body position and an angiogram image of a second body position of the blood vessel to be measured. The three-dimensional modeling unit 130 is configured to receive the angiogram image of the first body position and the angiogram image of the second body position transmitted by the coronary angiography extraction unit and to three-dimensionally model so as to obtain a three-dimensional coronary artery vascular model. The parametric measurement unit 140 is configured to receive the three-dimensional coronary artery vascular model transmitted by the three-dimensional modeling unit to obtain an average time $T_a$ taken for a contrast agent passing from an inlet to an outlet of a segment of blood vessel, and it is configured to obtain a time $T_{max}$ taken for the contrast agent passing from the inlet to the outlet of the segment of blood vessel in a maximum dilated state according to the three-dimensional coronary artery vascular model and hydrodynamic formulas. The coronary artery vascular evaluation parameters are obtained based on $T_a$, $T_{max}$ and/or $P_d$ and/or $P_a$.

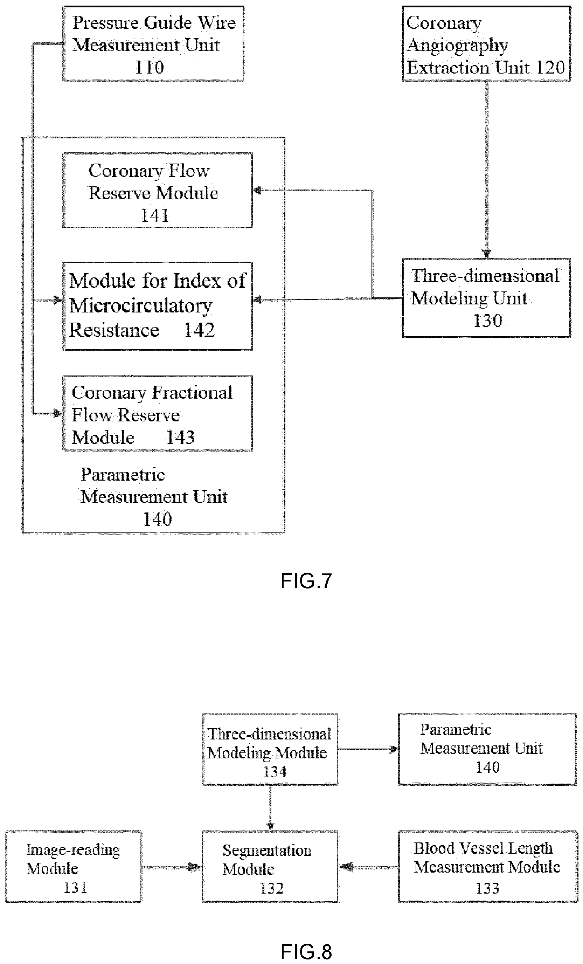

As shown in FIG. 7, in an embodiment of the present disclosure, the parametric measurement unit 140 comprises: a coronary flow reserve module 141, an module for index of microcirculatory resistance142, and/or a coronary fractional flow reserve module 143. Both the coronary flow reserve module 141 and the module for index of microcirculatory resistance 142 are connected to the three-dimensional modeling unit 130. Both the module for index of microcirculatory resistance 142 and the coronary fractional flow reserve module 143 are connected to the pressure guide wire measurement unit 110. The coronary flow reserve module 141 is configured to measure the coronary flow reserve CFR as the coronary artery blood flow increases from a resting state to a hyperemic state, $CFR= T_a/T_{max}$. The module for index of microcirculatory resistance 142 is configured to measure the index of microcirculatory resistance IMR, $IMR = P_d \times T_{max}$. The coronary fractional flow reserve module 143 is configured to measure the coronary fractional flow reserve FFR, $FFR=P_d/P_a$.

The parametric measurement unit 140 further comprises: a $T_1$ measurement module, a $T_2$ measurement module, and a CFR measurement module. All of them are connected to the three-dimensional modeling unit 130. Both the $T_1$ measurement module and $T_2$ measurement module are connected to the CFR measurement module.

As shown in FIG. 8, in an embodiment of the present disclosure, the three-dimensional modeling unit 130 comprises an image-reading module 131, a segmentation module 132, a blood vessel length measurement module 133, and a three-dimensional modeling module 134. The segmentation module 132 is connected to the image-reading module 131, the blood vessel length measurement module 133 and the three-dimensional modeling module 134. The image-reading module 131 is configured to read the angiogram image. The segmentation module 132 is configured to select one heartbeat cycle region of the coronary angiogram image. The blood vessel length measurement module 133 is configured to measure a length L of the blood vessel in the heartbeat cycle area, and transmit the length L of the blood vessel to the segmentation module 132. The three-dimensional modeling module 134 is configured to subject the coronary angiogram images selected by the segmentation module 132 to three-dimensional modeling so as to obtain the three-dimensional coronary vascular model.

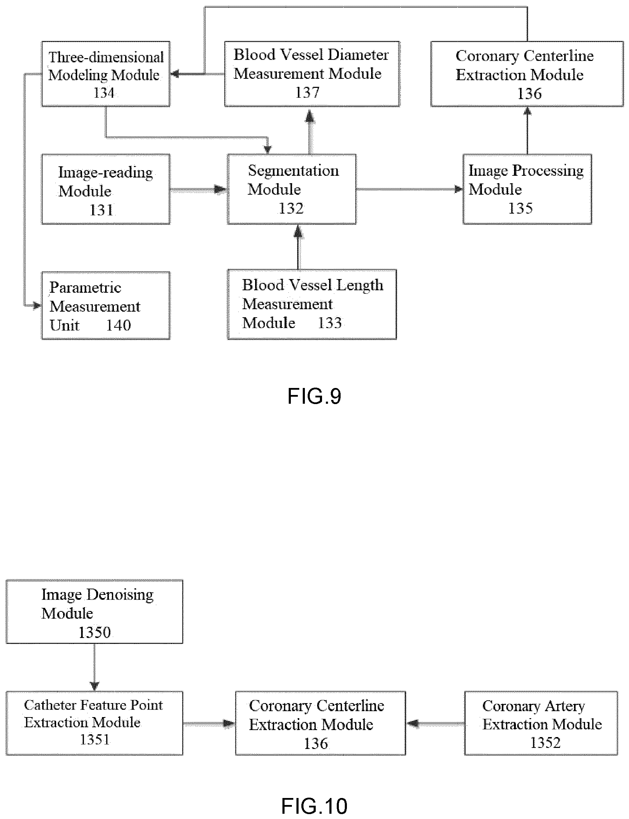

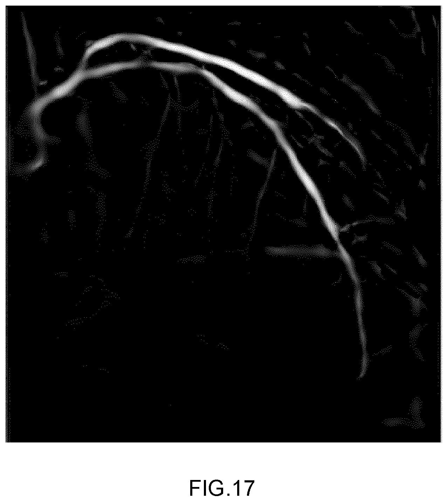

As shown in FIG. 9, in an embodiment of the present disclosure, the three-dimensional modeling unit 130 further comprises: an image processing module 135, a coronary centerline extraction module 136, and a blood vessel diameter measurement module 137. The image processing module 135 is connected to the coronary centerline extraction module 136. The three-dimensional modeling module 134 is connected to the coronary centerline extraction module 136 and the blood vessel diameter measurement module 137. The image processing module 135 is configured to receive the coronary angiogram images of at least two body positions transmitted by the segmentation module 132, and remove an interfering blood vessel from the coronary angiogram images to obtain the result image as shown in FIG. 17. The coronary centerline extraction module 136 is configured to extract the coronary centerline of each result image as shown in FIG. 17 along the extending direction of the coronary artery. The blood vessel diameter measurement module 137 is configured to measure diameter D of the blood vessel. The three-dimensional modeling module 134 is configured to project centerline and diameter of each coronary artery onto the three-dimensional space for three-dimensional modeling, thereby obtaining the three-dimensional coronary vascular model. The present disclosure realizes the synthesis of the three-dimensional coronary vascular model based on the coronary angiogram images, which fills up the gap in the industry and has positive effects on the field of medical technology.

In an embodiment of the present disclosure, an image denoising module 1350 is provided inside the image processing module 135 to denoise the coronary angiogram images, including static noise removal and dynamic noise removal. The denoising module 1350 removes interfering factors from the coronary angiogram images and improves the quality of image processing.

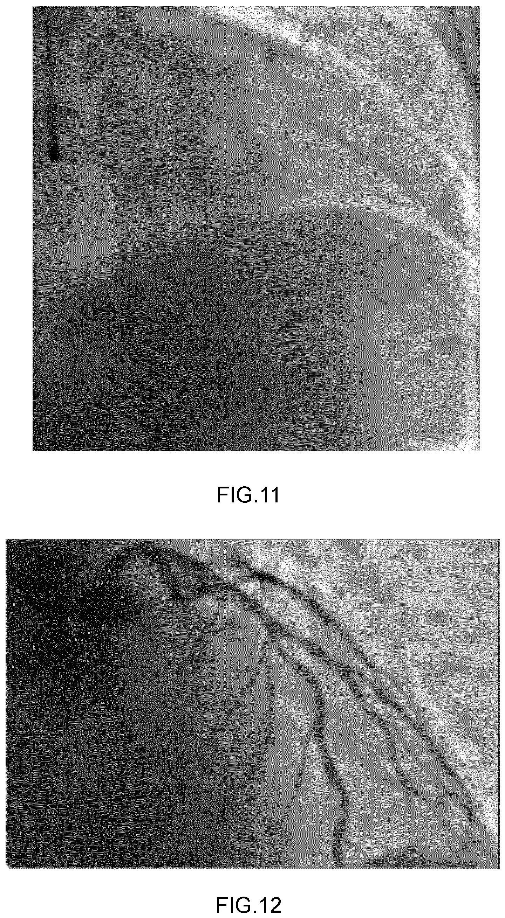

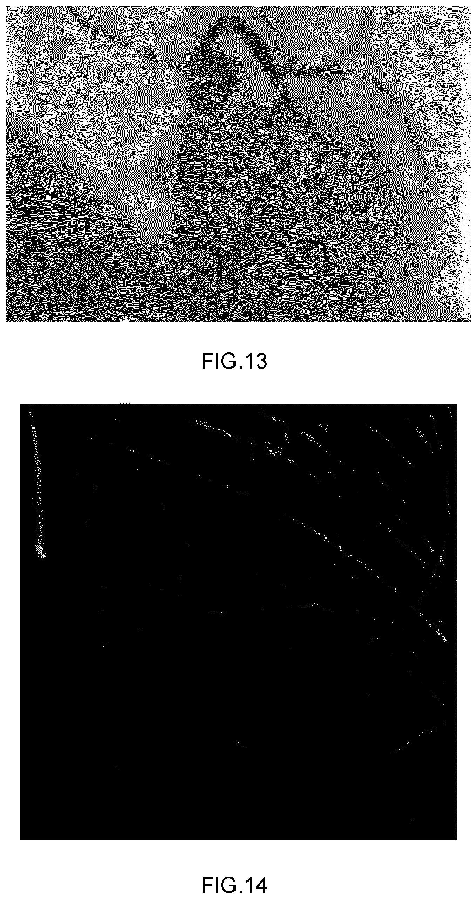

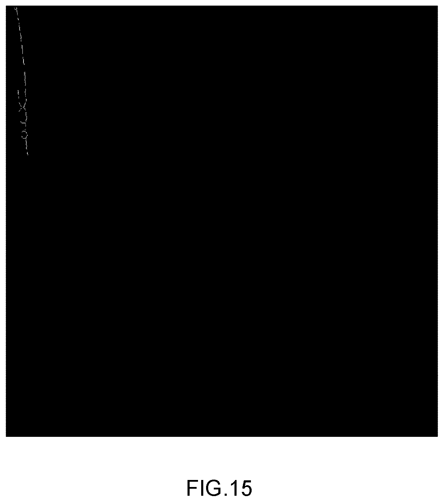

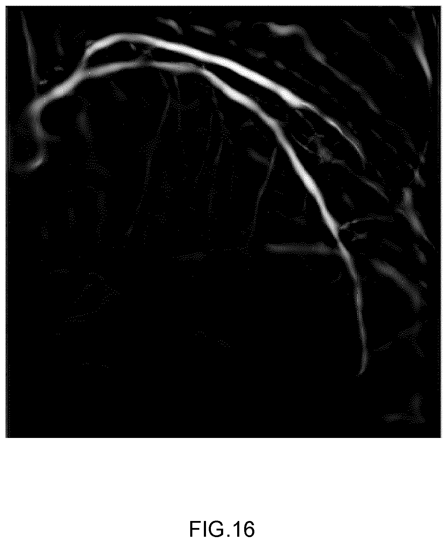

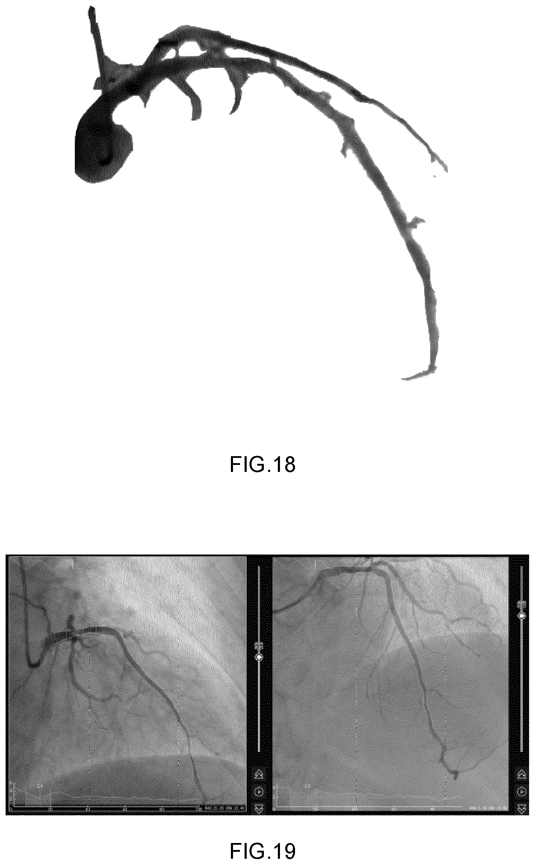

As shown in FIG. 10, in an embodiment of the present disclosure, the image processing module 135 is internally provided with a catheter feature point extraction module 1351 and a coronary artery extraction module 1352, both of which are connected to the coronary centerline extraction module 136. The catheter feature point extraction module 1351 is connected to the coronary artery extraction module 1352 and the image denoising module 1350. The catheter feature point extraction module 1351 is configured to define a first frame of a segmented image where the catheter appears as a reference image as shown in FIG. 11, and to define a k-th frame of the segmented image where the complete coronary artery appears as a target image as shown in FIG. 12 and FIG. 13, with k being a positive integer greater than 1. The target images as shown in FIG. 12 and FIG. 13 are enhanced to obtain the enhanced images as shown in FIG. 14 and FIG. 16. The target images shown in FIG. 12 and FIG. 13 are subtracted from the reference image shown in FIG. 11 to extract the feature point O of the catheter as shown in FIG. 15. The coronary artery extraction module 1352 is configured to subtract the reference image as shown in FIG. 11 from the target images as shown in FIG. 12 and FIG. 13, and to determine and extract a region of the coronary artery according to a positional relationship between each region in the enhanced target image shown in FIG. 16 and the catheter feature points, namely the image of region where the coronary artery locates as shown in FIG. 17. The image of region shown in FIG. 17 uses the catheter feature points as shown in FIG. 15 as seed points for dynamic growth to obtain the result image as shown in FIG. 18.

The image processing module 135 is also provided with a binarization processing module for binarizing the image to obtain a three-dimensional coronary vascular model.

The disclosure will be specifically described below in conjunction with specific embodiments:

Embodiment 1:

FIG. 19 shows coronary angiogram images of two body positions taken for a patient. A left image is an angiogram image with a body position angle being right anterior oblique RAO: 25° and a head position CRA: 23°. A right image is an angiogram image with a body position angle being right anterior oblique RAO: 3° and a head position CRA: 30°;

Place a pressure guide wire sensor on a distal end of the coronary artery of the patient (>5cm distanced from the opening of the guiding catheter). And inject a vasodilator into the blood vessels to make the blood vessels reach and maintain in the maximum dilated state (ensuring that the pressure guide wire sensor maintains in the same position before and after injection of the vasodilator). Then it can be measured via the pressure guide wire that $P_a$=87 Hg and $P_d$=86 mmHg were measured via.

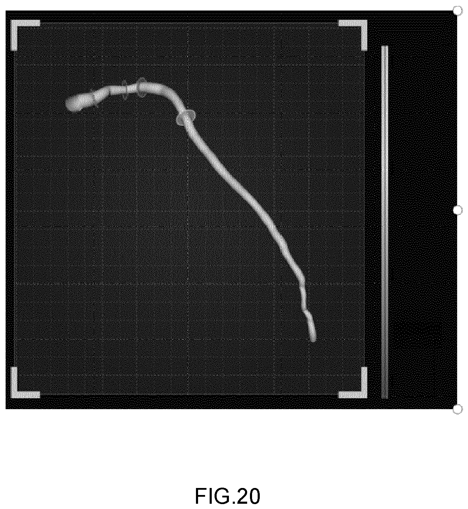

The L value of the blood vessel length of the three-dimensional coronary artery vascular model = 120 mm. The generated three-dimensional coronary vascular model is shown in FIG. 20; the diameter D of the blood vessel = 2~4 mm, $T_1=N_1/fps_1$=14/15=0.93s; $T_2=N_2/fps_2$=17/15=1.13s; $T_a$=(1.13+0.93)/2=1.03; and in the dilated state, V=L/(Nmax/$fps$)=120/(2/15) =900mm/s;

$$T_{max} = 120/900 = 0.13s;$$

$$CFR = T_a/T_{max} = 1.03/0.13 = 7.90;$$

thus,

$$IMR = 86 \times 0.13 = 9.46;$$

$$FFR = 86/87 = 0.99.$$

Comparative Embodiment 1:

The patient is the same as in Embodiment 1. Both Comparative Embodiment 1 and Embodiment 1 use the same coronary angiogram image taken for the same patient.

Place a pressure guide wire sensor on a distal end of the coronary artery of the patient (>5cm distanced from the opening of the guiding catheter), and then inject 3ml of normal saline into the blood vessel through the catheter. If the blood temperature was detected to return to the normal value, another 3 ml of normal saline would be injected into the blood vessel via the catheter. The above procedure was repeated for 3 times, and then $T_1$ was recorded as 0.83s. Deliver a vasodilator into the blood vessel to make the blood vessel reach and maintain in the maximum dilated state (ensuring that the pressure guide wire sensor was in the same position before and after the vasodilator injection). Then inject 3 ml of normal saline into the blood vessel through the catheter. If the blood temperature was detected to return to the normal value, another 3ml of normal saline was injected into the blood vessel through the catheter. The above procedure was repeated for 3 times, and then $T_2$ is recorded as 0.11s. Then a pressure at a distal end of the coronary artery was measured as $P_d$=84 mmHg, and a pressure at the coronary artery inlet was measured as $P_a$=85 mmHg;

$$CFR=0.83/0.11=7.54;$$

$$IMR=P_d \times T_2=84 \times 0.11=9.24;$$

$$FFR=84/85=0.988.$$

By comparing Embodiment 1 and Comparative Embodiment 1, the difference was within 0.5. It can be seen that the IMR measurement results are basically the same. Therefore, the measurement results of Embodiment 1 are accurate. Further, the embodiments of the disclosure use a pressure guide wire to measure the pressure at the distal end without using normal saline, and measure $T_1$ and $T_2$ by a three-dimensional vascular model; and the IMR measurement is realized through the angiogram image. It fills up the gap in the industry and makes the operation simpler, and also realizes the FFR measurement without normal saline, solving the problem that the position of pressure guide wire sensor is difficult to control under the impulse of the normal saline, and solving the problem of inaccurate measurement of the pressure at the distal end. Moreover, there is no need for multiple injections of normal saline, and the procedure is thus convenient and quick.

The present disclosure provides a coronary artery analysis system comprising the above apparatus for conveniently measuring the coronary artery vascular evaluation parameters.

The present disclosure provides a computer storage medium, and the aforementioned method for conveniently measuring the coronary artery vascular evaluation parameters is implemented when a computer program is executed by a processor.

A person skilled in the art knows that various aspects of the present disclosure can be implemented as a system, a method, or a computer program product. Therefore, each aspect of the present disclosure can be specifically implemented in the following forms, namely: complete hardware implementation, complete software implementation (including firmware, resident software, microcode, etc.), or a combination of hardware and software implementations, which here can be collectively referred to as "circuit", "module" or "system". In addition, in some embodiments, various aspects of the present disclosure may also be implemented in the form of a computer program product in one or more computer-readable media, and the computer-readable medium contains computer-readable program code. Implementation of a method and/or a system of embodiments of the present disclosure may involve performing or completing selected tasks manually, automatically, or a combination thereof.

For example, hardware for performing selected tasks according to the embodiment(s) of the present disclosure may be implemented as a chip or a circuit. As software, selected tasks according to the embodiment(s) of the present disclosure can be implemented as a plurality of software instructions executed by a computer using any suitable operating system. In the exemplary embodiment(s) of the present disclosure, a data processor performs one or more tasks according to the exemplary embodiment(s) of a method and/or system as described herein, such as a computing platform for executing multiple instructions. Optionally, the data processor comprises a volatile memory for storing instructions and/or data, and/or a non-volatile memory for storing instructions and/or data, for example, a magnetic hard disk and/or movable medium. Optionally, a network connection is also provided. Optionally, a display and/or user input device, such as a keyboard or mouse, are/is also provided.

Any combination of one or more computer readable media can be utilized. The computer-readable medium may be a computer-readable signal medium or a computer-readable storage medium. The computer-readable storage medium may be, for example, but not limited to, an electrical, magnetic, optical, electromagnetic, infrared, or semiconductor system, apparatus, or device, or any combination of the above. More specific examples (non-exhaustive list) of computer-readable storage media would include the following:

Electrical connection with one or more wires, portable computer disk, hard disk, random access memory (RAM), read only memory (ROM), erasable programmable read only memory (EPROM or flash memory), optical fiber, portable compact disk read only memory (CD-ROM), optical storage device, magnetic storage device, or any suitable combination of the above. In this document, the computer-readable storage medium can be any tangible medium that contains or stores a program, and the program can be used by or in combination with an instruction execution system, apparatus, or device.

The computer-readable signal medium may include a data signal propagated in baseband or as a part of a carrier wave, which carries computer-readable program code. This data signal for propagation can take many forms, including but not limited to electromagnetic signals, optical signals, or any suitable combination of the above. The computer-readable signal medium may also be any computer-readable medium other than the computer-readable storage medium. The computer-readable medium can send, propagate, or transmit a program for use by or in combination with the instruction execution system, apparatus, or device.

The program code contained in the computer-readable medium can be transmitted by any suitable medium, including, but not limited to, wireless, wired, optical cable, RF, etc., or any suitable combination of the above.

For example, any combination of one or more programming languages can be used to write computer program codes for performing operations for various aspects of the present disclosure, including object-oriented programming languages such as Java, Smalltalk, C++, and conventional process programming languages, such as "C" programming language or similar programming language. The program code can be executed entirely on a user's computer, partly on a user's computer, executed as an independent software package, partly on a user's computer and partly on a remote computer, or entirely on a remote computer or server. In the case of a remote computer, the remote computer can be connected to a user's computer through any kind of network including a local area network (LAN) or a wide area network (WAN), or it can be connected to an external computer (for example, connected through Internet provided by an Internet service provider).

It should be understood that each block of the flowcharts and/or block diagrams and combinations of blocks in the flowcharts and/or block diagrams can be implemented by computer program instructions. These computer program instructions can be provided to the processor of general-purpose computers, special-purpose computers, or other programmable data processing devices to produce a machine, which produces a device that implements the functions/actions specified in one or more blocks in the flowcharts and/or block diagrams when these computer program instructions are executed by the processor of the computer or other programmable data processing devices.

It is also possible to store these computer program instructions in a computer-readable medium. These instructions make computers, other programmable data processing devices, or other devices work in a specific manner, so that the instructions stored in the computer-readable medium generate an article of manufacture comprising instructions for implementation of the functions/actions specified in one or more blocks in the flowcharts and/or block diagrams.

Computer program instructions can also be loaded onto a computer (for example, a coronary artery analysis system) or other programmable data processing equipment to facilitate a series of operation steps to be performed on the computer, other programmable data processing apparatus or other apparatus to produce a computer-implemented process, which enable instructions executed on a computer, other programmable device, or other apparatus to provide a process for implementing the functions/actions specified in the flowcharts and/or one or more block diagrams.

The above specific examples of the present disclosure further describe the purpose, technical solutions and beneficial effects of the present disclosure in detail. It should be understood that the above are only specific embodiments of the present disclosure and are not intended to limit the present disclosure. Within the spirit and principle of the present disclosure, any modification, equivalent replacement, improvement, etc. shall be included in the protection scope of the present disclosure.

**Claims**

1. A method for conveniently measuring coronary artery vascular evaluation parameters, **characterized by** comprising:

   measuring a pressure $P_d$ at a distal end of coronary artery stenosis and/or a pressure $P_a$ at a coronary artery inlet via a pressure guide wire;
   performing coronary angiography for a blood vessel to be measured;
   selecting an angiogram image of a first body position and an angiogram image of a second body position of the blood vessel to be measured;
   selecting a segment of blood vessel from a proximal end to a distal end of the coronary artery for segmentation, and obtaining a three-dimensional coronary artery vascular model by three-dimensional modeling based on the angiogram image of the first body position and the angiogram image of the second body position;
   injecting a contrast agent, and obtaining an average time $T_a$ taken for the contrast agent passing from an inlet to an outlet of the segment of blood vessel according to the three-dimensional coronary artery vascular model;
   obtaining a time $T_{max}$ taken for the contrast agent passing from the inlet to the outlet of the segment of blood vessel in a maximum dilated state according to the three-dimensional coronary artery vascular model and hydrodynamic formulas;
   obtaining coronary artery vascular evaluation parameters based on $T_a$, $T_{max}$ and/or $P_d$ and/or $P_a$.

**2.** The method for conveniently measuring coronary artery vascular evaluation parameters according to claim 1, **characterized in that** the coronary artery vascular evaluation parameters comprise coronary flow reserve CFR and an index of microcirculatory resistance IMR as coronary artery blood flow increases from a resting state to a hyperemic state.

**3.** The method for conveniently measuring coronary artery vascular evaluation parameters according to claim 2, **characterized in that** the CFR= $T_a/T_{max}$; and/or
the IMR = $P_d \times T_{max}$.

**4.** The method for conveniently measuring coronary artery vascular evaluation parameters according to any one of claims 1 to 3, **characterized in that** obtaining an average time $T_a$ taken for the contrast agent passing from an inlet to an outlet of the segment of blood vessel comprises:
obtaining a time $T_1$ taken for the contrast agent passing from an inlet to an outlet of the segment of blood vessel within the angiogram image of the first body position and obtaining a time $T_2$ taken for the contrast agent passing from an inlet to an outlet of the segment of blood vessel within the angiogram image of the second body position, $$T_a = \frac{(T_1 + T_2)}{2}.$$

**5.** The method for conveniently measuring coronary artery vascular evaluation parameters according to claim 4, **characterized in that** the time $T_1$ and the time $T_2$ are calculated according to a ratio of the number of frames of partial area images divided by a heartbeat cycle area to the number of frames transmitted per second.

**6.** The method for conveniently measuring coronary artery vascular evaluation parameters according to any one of claims 1 to 3, **characterized in that** obtaining a time $T_{max}$ taken for the contrast agent passing from the inlet to the outlet of the segment of blood vessel in the maximum dilated state, comprises:

measuring a length L of the selected segment of the blood vessel;
deriving a blood flow velocity V in a dilated state by means of the hydrodynamic calculating method according to the three-dimensional coronary artery vascular model, $P_a$ and $P_d$;
obtaining the time $T_{max}$ taken for the contrast agent passing from the inlet to the outlet of the segment of blood vessel in the maximum dilated state according to the formula $T_{max} = L/V$.

**7.** The method for conveniently measuring coronary artery vascular evaluation parameters according to claim 6, **characterized in that** deriving a blood flow velocity V in the dilated state by means of the hydrodynamic calculating method according to the three-dimensional coronary artery vascular model, $P_a$ and $P_d$ comprises:

obtaining the diameter Dt of the segment of blood vessel at a time interval of t;
obtaining the pressure $P_d$ at the distal end of coronary artery stenosis and the pressure $P_a$ at the coronary artery inlet via the pressure guide wire at the time interval of t;
calculating a FFRt value at the time interval of t according to Dt, $P_a$, and $P_d$, and obtaining a blood flow velocity Vt by inverse calculation according to the definition of FFR;
injecting a vasodilator and measuring the FFR value in the dilated state; comparing the FFR value in the dilated state with the real-time FFRt value to obtain the blood flow velocity Vt correspondingly, namely the blood flow velocity V in the dilated state.

**8.** The method for conveniently measuring coronary artery vascular evaluation parameters according to any one of claims 1 to 3, **characterized in that** an angle between the first body position and the second body position is greater than 30°

**9.** The method for conveniently measuring coronary artery vascular evaluation parameters according to any one of claims 1 to 3, **characterized in that** obtaining a three-dimensional coronary artery vascular model by three-dimensional modeling based on the angiogram image of the first body position and the angiogram image of the second body position comprises:

removing an interfering blood vessel from the angiogram image of the first body position and the angiogram image of the second body position to obtain a result image;
extracting a centerline and diameter of the coronary artery from each result image along an extension direction of the coronary artery;
projecting the centerline and diameter of each coronary artery onto a three-dimensional space for three-dimensional modeling to obtain a three-dimensional coronary artery vascular model.

**10.** An apparatus for conveniently measuring coronary artery vascular evaluation parameters, for use in the method for conveniently measuring coronary artery vascular evaluation parameters according to any one of claims 1 to 9, **characterized by** comprising:
a pressure guide wire measurement unit, a coronary angiography extraction unit, a three-dimensional modeling unit and a parametric measurement unit; the coronary angiography extraction unit being connected to the three-dimensional modeling unit; the parametric measurement unit being connected to the pressure guide wire measurement unit and the three-dimensional modeling unit;

the pressure guide wire measurement unit being configured to measure a pressure $P_d$ at a distal end of coronary artery stenosis and a pressure $P_a$ at a coronary artery inlet via the pressure guide wire;

the coronary angiography extraction unit being configured to select an angiogram image of a first body position and an angiogram image of a second body position of a blood vessel to be measured;

the three-dimensional modeling unit being configured to receive the angiogram image of the first body position and the angiogram image of the second body position transmitted by the coronary angiography extraction unit and to three-dimensionally model so as to obtain a three-dimensional coronary artery vascular model;

the parametric measurement unit being configured to receive the three-dimensional coronary artery vascular model transmitted by the three-dimensional modeling unit to obtain an average time $T_a$ taken for a contrast agent passing from an inlet to an outlet of a segment of blood vessel; and obtaining a time $T_{max}$ taken for the contrast agent passing from the inlet to the outlet of the segment of blood vessel in a maximum dilated state according to the three-dimensional coronary artery vascular model and hydrodynamic formulas; obtaining coronary artery vascular evaluation parameters based on $T_a$, $T_{max}$ and/or $P_d$ and/or $P_a$.

11. The apparatus for conveniently measuring the coronary artery vascular evaluation parameters according to claim 10, **characterized in that** the parametric measurement unit comprises: a coronary flow reserve module, a module for index of microcirculatory resistance, and/or a coronary fractional flow reserve module; the coronary flow reserve module and the module for index of microcirculatory resistance being connected to the three-dimensional modeling unit; the module for index of microcirculatory resistance and the coronary fractional flow reserve module being connected to the pressure guide wire measurement unit;

the coronary flow reserve module being configured to measure the coronary flow reserve CFR as the coronary artery blood flow increases from a resting state to a hyperemic state, CFR= $T_a/T_{max}$;

the module for index of microcirculatory resistance being configured to measure the index of microcirculatory resistance IMR, IMR = $P_d \times T_{max}$;

the coronary fractional flow reserve module being configured to measure the coronary fractional flow reserve FFR, FFR=$P_d/P_a$.

12. A coronary artery analysis system, **characterized by** comprising the apparatus for conveniently measuring the coronary artery vascular evaluation parameters according to claim 10 or 11.

13. A computer storage medium, **characterized in that** the method for conveniently measuring the coronary artery vascular evaluation parameters according to any one of claims 1 to 9 is implemented when a computer program is executed by a processor.

S100 — Measuring a pressure $P_d$ at a distal end of coronary artery stenosis and/or a pressure $P_a$ at a coronary artery inlet via a pressure guide wire;

S200 — Performing coronary angiography for a blood vessel to be measured;

S300 — Selecting, in a resting state, an angiogram image of a first body position and an angiogram image of a second body position of the blood vessel to be measured;

S400 — Selecting a segment of blood vessel from a proximal end to a distal end of the coronary artery for segmentation, and obtaining a three-dimensional coronary artery vascular model by three-dimensional modeling based on the angiogram image of the first body position and the angiogram image of the second body position;

S500 — Injecting a contrast agent, and obtaining an average time $T_a$ taken for the contrast agent passing from an inlet to an outlet of the segment of blood vessel according to the three-dimensional coronary artery vascular model;

S600 — Obtaining a time $T_{max}$ taken for the contrast agent passing from the inlet to the outlet of the segment of blood vessel in a maximum dilated state according to the three-dimensional coronary artery vascular model and hydrodynamic formulas;

S700 — Obtaining coronary artery vascular evaluation parameters based on $T_a$, $T_{max}$ and/or $P_d$ and/or $P_a$.

FIG.1

S100 — Measuring a pressure $P_d$ at a distal end of coronary artery stenosis and/or a pressure $P_a$ at a coronary artery inlet via a pressure guide wire;

S110 — Injecting a contrast agent into the blood vessel

S200 — Performing coronary angiography for a blood vessel to be measured;

S300 — Selecting, in a resting state, an angiogram image of a first body position and an angiogram image of a second body position of the blood vessel to be measured;

S400 — Selecting a segment of blood vessel from a proximal end to a distal end of the coronary artery for segmentation, and obtaining a three-dimensional coronary artery vascular model by three-dimensional modeling based on the angiogram image of the first body position and the angiogram image of the second body position;

S500 — Injecting a contrast agent, and obtaining an average time $T_a$ taken for the contrast agent passing from an inlet to an outlet of the segment of blood vessel according to the three-dimensional coronary artery vascular model;

S600 — Obtaining a time $T_{max}$ taken for the contrast agent passing from the inlet to the outlet of the segment of blood vessel in a maximum dilated state according to the three-dimensional coronary artery vascular model and hydrodynamic formulas;

S700 — Obtaining coronary artery vascular evaluation parameters based on $T_a$, $T_{max}$ and/or $P_d$ and/or $P_a$.

FIG.2

S410 — Removing an interfering blood vessel from the angiogram image of the first body position and the angiogram image of the second body position to obtain a result image;

S420 — Extracting a centerline and diameter of the coronary artery from each result image along an extension direction of the coronary artery;

S430 — Projecting the centerline and diameter of each coronary artery onto a three-dimensional space for three-dimensional modeling to obtain a three-dimensional coronary artery vascular model.

## FIG.3

S610 — Measuring a length L of the selected segment of the blood vessel;

S620 — Deriving a blood flow velocity V in a dilated state from the hydrodynamic calculating method according to the three-dimensional coronary artery vascular model, $P_a$ and $P_d$;

S630 — Obtaining the time $T_{max}$ taken for the contrast agent passing from the inlet to the outlet of the segment of blood vessel in the maximum dilated state according to the formula $T_{max} = L/V$.

## FIG.4

S621 — Obtaining the diameter $D_t$ of the segment of blood vessel at a time interval of t;

S622 — Obtaining the pressure $P_d$ at the distal end of coronary artery stenosis and the pressure $P_a$ at the coronary artery inlet via the pressure guide wire at the time interval of t;

S623 — Calculating a $FFR_t$ value at the time interval of t according to $D_t$, $P_a$, and $P_d$, and obtaining a blood flow velocity $V_t$ by inverse calculation according to the definition of FFR;

S624 — Injecting a vasodilator and measuring the FFR value in the dilated state; comparing the FFR value in the dilated state with the real-time $FFR_t$ value to obtain the blood flow velocity $V_t$ correspondingly, namely the blood flow velocity V in the dilated state. The disclosure reduces the injection volume and the number of injections of the vasodilator, which therefore is safer and more reliable.

## FIG.5

| Pressure Guide Wire Measurement Unit 110 | Coronary Angiography Extraction Unit 120 |
|---|---|
| Parametric Measurement Unit 140 | Three-dimensional Modeling Unit 130 |

FIG.6

| Pressure Guide Wire Measurement Unit 110 | | Coronary Angiography Extraction Unit 120 |

Coronary Flow Reserve Module 141

Module for Index of Microcirculatory Resistance 142

Three-dimensional Modeling Unit 130

Coronary Fractional Flow Reserve Module 143

Parametric Measurement Unit 140

FIG.7

Three-dimensional Modeling Module 134

Parametric Measurement Unit 140

Image-reading Module 131

Segmentation Module 132

Blood Vessel Length Measurement Module 133

FIG.8

```
┌─────────────────┐   ┌─────────────────────┐   ┌──────────────────┐
│ Three-dimensional│  │ Blood Vessel Diameter│  │ Coronary Centerline│
│ Modeling Module │◄─│ Measurement Module  │  │ Extraction Module │
│      134        │   │       137           │  │       136         │
└─────────────────┘   └─────────────────────┘   └──────────────────┘

┌─────────────────┐   ┌─────────────────┐   ┌──────────────────┐
│ Image-reading   │  │ Segmentation    │  │ Image Processing │
│ Module          │─►│ Module          │─►│ Module           │
│      131        │   │      132        │  │      135         │
└─────────────────┘   └─────────────────┘   └──────────────────┘

┌─────────────────┐   ┌─────────────────┐
│ Parametric      │  │ Blood Vessel Length│
│ Measurement     │  │ Measurement     │
│ Unit      140   │  │ Module    133   │
└─────────────────┘   └─────────────────┘
```

FIG.9

```
┌─────────────────┐
│ Image Denoising │
│ Module          │
│      1350       │
└─────────────────┘

┌──────────────────┐   ┌──────────────────┐   ┌──────────────────┐
│ Catheter Feature Point│ │ Coronary Centerline│ │ Coronary Artery  │
│ Extraction Module│─►│ Extraction Module │◄─│ Extraction Module │
│      1351        │   │      136          │  │      1352         │
└──────────────────┘   └──────────────────┘   └──────────────────┘
```

FIG.10

FIG.11

FIG.12

FIG.13

FIG.14

FIG.15

FIG.16

FIG.17

FIG.18

FIG.19

FIG.20

## INTERNATIONAL SEARCH REPORT

| International application No. |
|---|
| **PCT/CN2019/115043** |

**A.    CLASSIFICATION OF SUBJECT MATTER**

A61B 5/026(2006.01)i

According to International Patent Classification (IPC) or to both national classification and IPC

**B.    FIELDS SEARCHED**

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

WPI, EPODOC, CNPAT, CNKI: 润迈德, 润心, 龚艳君, 刘广志, 王志婷, 顾逸凡, 王之元, 霍勇, 易铁慈, 李建平, 冠脉, 冠状动脉, 血管, 评定, 评估, 血流储备, 微循环阻力, 血压, 压力, 时间, 造影, 图像, 模型, 充血, 扩张, coronary, artery, vessel, image?, model?, pressure?, T, time, CFR, FFR, IMR

**C.    DOCUMENTS CONSIDERED TO BE RELEVANT**

| Category* | Citation of document, with indication, where appropriate, of the relevant passages | Relevant to claim No. |
|---|---|---|
| PX | CN 110367965 A (SUZHOU RAINMED MEDICAL TECHNOLOGY CO., LTD.) 25 October 2019 (2019-10-25)<br>claims 1-13, description paragraphs [0009]-[0150], figures 1-19 | 1-13 |
| A | CN 108550189 A (SUZHOU RAINMED MEDICAL TECHNOLOGY CO., LTD.) 18 September 2018 (2018-09-18)<br>description, paragraphs [0006]-[0054] | 1-13 |
| A | CN 102525443 A (SIEMENS AKTIENGESELLSCHAFT) 04 July 2012 (2012-07-04)<br>entire document | 1-13 |
| A | CN 107978371 A (BODONG MEDICAL IMAGING TECHNOLOGY (SHANGHAI) CO., LTD.) 01 May 2018 (2018-05-01)<br>entire document | 1-13 |
| A | CN 105326486 A (SHANGHAI JIAO TONG UNIVERSITY et al.) 17 February 2016 (2016-02-17)<br>entire document | 1-13 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 May 2020** | **26 May 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

# EP 4 026 492 A1

| | | International application No. |
|---|---|---|
| **INTERNATIONAL SEARCH REPORT** | | **PCT/CN2019/115043** |

**C.    DOCUMENTS CONSIDERED TO BE RELEVANT**

| Category* | Citation of document, with indication, where appropriate, of the relevant passages | Relevant to claim No. |
|---|---|---|
| A | CN 107730540 A (QUANJING HENGSHENG (BEIJING) SCIENCE AND TECHNOLOGY CO., LTD.) 23 February 2018 (2018-02-23) entire document | 1-13 |
| A | CN 110120031 A (SICHUAN JINJIANG ELECTRONIC SCIENCE AND TECHNOLOGY CO., LTD.) 13 August 2019 (2019-08-13) entire document | 1-13 |
| A | CN 108992057 A (HANGZHOU SHENGSHI TECHNOLOGY CO., LTD.) 14 December 2018 (2018-12-14) entire document | 1-13 |
| A | US 2015250395 A1 (KABUSHIKI KAISHA TOSHINA et al.) 10 September 2015 (2015-09-10) entire document | 1-13 |

<table>
<thead>
<tr><th colspan="2">INTERNATIONAL SEARCH REPORT<br>Information on patent family members</th><th colspan="2">International application No.<br>PCT/CN2019/115043</th></tr>
</thead>
</table>

| Patent document cited in search report | | | Publication date (day/month/year) | Patent family member(s) | | | Publication date (day/month/year) |
|---|---|---|---|---|---|---|---|
| CN | 110367965 | A | 25 October 2019 | CN | 110393516 | A | 01 November 2019 |
| | | | | CN | 110384493 | A | 29 October 2019 |
| | | | | CN | 110522439 | A | 03 December 2019 |
| | | | | CN | 110384494 | A | 29 October 2019 |
| CN | 108550189 | A | 18 September 2018 | WO | 2019210553 | A1 | 07 November 2019 |
| CN | 102525443 | A | 04 July 2012 | CN | 102525443 | B | 11 May 2016 |
| | | | | US | 2016066800 | A1 | 10 March 2016 |
| | | | | US | 9999361 | B2 | 19 June 2018 |
| | | | | US | 2012072190 | A1 | 22 March 2012 |
| | | | | US | 9119540 | B2 | 01 September 2015 |
| CN | 107978371 | A | 01 May 2018 | | None | | |
| CN | 105326486 | A | 17 February 2016 | JP | 2019213903 | A | 19 December 2019 |
| | | | | WO | 2017097073 | A1 | 15 June 2017 |
| | | | | JP | 6611959 | B2 | 27 November 2019 |
| | | | | US | 2018344173 | A1 | 06 December 2018 |
| | | | | JP | 2018535816 | A | 06 December 2018 |
| | | | | CN | 105326486 | B | 25 August 2017 |
| | | | | DE | 112016005603 | T5 | 25 October 2018 |
| CN | 107730540 | A | 23 February 2018 | US | 2019110776 | A1 | 18 April 2019 |
| CN | 110120031 | A | 13 August 2019 | | None | | |
| CN | 108992057 | A | 14 December 2018 | | None | | |
| US | 2015250395 | A1 | 10 September 2015 | US | 9877657 | B2 | 30 January 2018 |
| | | | | JP | 2015167790 | A | 28 September 2015 |
| | | | | JP | 6262027 | B2 | 17 January 2018 |
| | | | | US | 2018098706 | A1 | 12 April 2018 |
| | | | | JP | 2019147004 | A | 05 September 2019 |
| | | | | JP | 2018064967 | A | 26 April 2018 |
| | | | | JP | 6530043 | B2 | 12 June 2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910835038